# EUROPÄISCHE PATENTSCHRIFT

(11) **EP 0 910 601 B1**
(45) Veröffentlichungstag und Bekanntmachung des Hinweises auf die Patenterteilung: **08.03.2000**
(21) Anmeldenummer: 97930389.8
(22) Anmeldetag: 24.06.1997
(51) Int. Cl.: C08G 69/48, C12N 11/08, C07K 17/08

(54) **AZLACTON-DERIVATISIERTE POLYAMIDE**
AZLACTONE-DERIVATIZED POLYAMIDES
POLYAMIDES DERIVES D'AZLACTONE

(30) Priorität: 06.07.1996 DE 19627302
(43) Veröffentlichungstag der Anmeldung: 28.04.1999
(73) Patentinhaber: MERCK PATENT GmbH, 64293 Darmstadt (DE)
(72) Erfinder: MÜLLER, Egbert, D-64390 Erzhausen (DE); SEILER, Anja, D-64846 Gro -Zimmern (DE)
(86) Internationale Anmeldenummer: EP9703300
(87) Internationale Veröffentlichungsnummer: WO9801496

(56) Entgegenhaltungen:
- EP-A- 0 392 783
- US-A- 4 737 560
- US-A- 5 408 002

## Beschreibung

Die Erfindung betrifft azlacton-derivatisierte Polyamide, sowie Formkörper enthaltend azlacton-derivatisierte Polyamide, sowie Verfahren zu ihrer Herstellung.

Azlacton-derivatisierte Trägermaterialien sind aus der Literatur bekannt; beispielsweise offenbart US 4,737,560 vernetzte Trägermaterialien, die Azlactongruppen enthalten. EP 0392 783 offenbart Verfahren zur Pfropfpolymerisation von Grundpolymeren, wobei die aufgepfropften Seitenketten Aclactongruppen enthalten. Diese Trägermaterialien sind zur Bindung von Proteinen geeignet, soweit die Azlactongruppen für hochmolekulare Verbindungen hinreichend zugänglich sind. Poröse Membranen aus Polyamiden weisen hervorragende Flußeigenschaften auf; Membranen aus Polyamiden können auch mit Poren hergestellt werden, deren innere Oberflächen für hochmolekulare Verbindungen gut zugänglich sind. Polyamide weisen außerdem geringe unspezifische Bindungen gegenüber Proteinen auf.

Die Patentanmeldung DE 195 01 726.9 (WO 96/22 316) offenbart derivatisierte Polyamidmembranen, die als Blockpolymere vorliegen. Die aufpolymerisierten Monomeren können auch zu Azlactongruppen umgesetzt werden. Die Herstellung dieser azlactonhaltigen Polyamidmembranen ist relativ aufwendig, da zunächst ein polymerisierbares Derivat des Polyamids hergestellt werden muß, dann muß das Blockpolymere hergestellt werden, wobei die dabei verwendeten Monomeren Vorläuferverbindungen für die Azlactongruppe enthalten. Schließlich müssen die Vorläuferverbindungen in die Azlactongruppe überführt werden. Es besteht also die Aufgabe, ein vereinfachtes Verfahren zur Herstellung von azlacton-derivatisierten Polyamiden bereitzustellen.

Es wurde gefunden, daß Aminogruppen, die üblicherweise terminal in Polyamiden vorliegen, mit Vinylazlactonderivaten umgesetzt werden können, wobei die Vinylgruppierung mit der Aminogruppe reagiert und somit auf einfache Art und Weise ein azlacton-derivatisiertes Polyamid entsteht. Da die Azlactongruppe bekanntermaßen mit Aminogruppen reagiert, ist diese Reaktionsmöglichkeit unerwartet. Soweit die Polyamide, die als Grundpolymer verwendet werden, Carboxylgruppen enthalten, können diese Carboxylgruppen nach dem in DE 196 24 813.2 (PCT/EP97/ 02 768) offenbarten Verfahren zunächst mit Diaminoverbindungen umgesetzt werden, wobei zusätzliche Aminogruppen bereitgestellt werden.

Gegenstand der Erfindung sind azlacton-derivatisierte Polyamide erhältlich durch Umsetzung von Polyamiden mit einer Lösung enthaltend eine Azabicycloverbindung und ein Vinylazlactonderivat der Formel I, 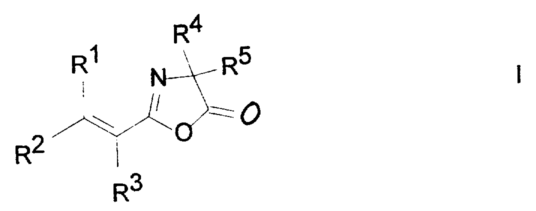 worin
- R¹, R² und R³: unabhängig voneinander
H oder CH₃;
- R⁴ und R⁵: unabhängig voneinander
H oder C₁- bis C₅- Alkyl
bedeuten.

Bevorzugt bedeuten R¹, R² und R³ H, sowie R⁴ und R⁵ Methyl.

Gegenstand der Erfindung ist die Verwendung der azlacton-derivatisierten Polyamide für die Bindung von aminogruppenhaltigen Verbindungen. Geeignete aminogruppenhaltige Verbindungen sind beispielsweise Proteine, darunter auch Enzyme und Antikörper.

Gegenstand der Erfindung sind Trennmaterialien erhältlich durch Umsetzung eines erfindungsgemäßen azlacton-derivatisierten Polyamides mit einem aminogruppenhaltigen Separationseffektor. Geeignete aminogruppenhaltige Separationseffektoren sind beispielsweise Antikörper und andere Affinitätsliganden für die Affinitätschromatographie. Andere Separationseffektoren sind dem Fachmann bekannt.

Gegenstand der Erfindung sind immobilisierte Enzyme erhältlich durch Umsetzung eines erfindungsgemäßen azlacton-derivatisierten Polyamides mit einem Enzym.

Als Basispolymer geeignetete Polyamide sind dem Fachmann bekannt und sind auch kommerziell erhältlich. Dazu gehören z.B. die unter dem Handelsnamen NYLON® bekannten Polymere, z.B. NYLON® 66 und NYLON® 6. Poröse oder unporöse Formkörper bestehend aus derartigen Polyamiden sind ebenfalls bekannt und auch kommerziell erhältlich; dazu gehören beispielsweise perlförmige Formkörper, Membranen, Schläuche, Hohlfasermembranen, Schwämme. Die Umsetzung derartiger Formkörper ist bevorzugt, da unter den Reaktionsbedingungungen, wie sie in DE 195 01 726.9 und DE 196 24 813.2 verwendet werden (Reaktionstemperatur unter 80 °C), deren Form erhalten bleibt, während andere Verfahren zur Derivatisierung von Polyamid in der Schmelze oder in Lösung ausgeführt werden.

Die erfindungsgemäße Reaktionsfolge wird bevorzugterweise zwischen 0 und 70 °C, insbesondere zwischen 10 und 60 °C, ausgeführt, d.h. bei Temperaturen unterhalb des Schmelzpunktes des Ausgangspolyamids. Da das Ausgangspolyamid nicht gelöst werden muß, kann die erfindungsgemäße Reaktionsfolge in bevorzugter Weise auch auf Formkörper aus Polyamiden angewandt werden, ohne daß deren Form eine nennenswerte Änderung erfährt. Die Reaktion wird durch Azabicyclo-Verbindungen, z.B. 1,8-Diazabicyclo[5,4,0]undec-7-en (DBU), 1,5-Diazabicyclo[4,3,0]non-5-en (DBN) oder 1,5,7-Triazabicyclo[4,4,0]dec-5-en (TBD) katalysiert. Das Vinylazlactonderivat wird zusammen mit dem Katalysator in einem gegenüber dem Azlactonderivat inerten Lösungsmittel gelöst. Bevorzugt sind Lösungsmittel, die das Polyamid nicht auflösen; z.B. Dimethylformamid (DMF) oder Dimethylsulfoxid (DMSO).

### Beispiele

Die folgenden Beispiele sollen die Erfindung näher erläutern.

Im folgenden wird unter Raumtemperatur eine Temperatur zwischen 15 und 30 °C verstanden.

### Beispiel 1: Umsetzung einer Polyamidhohlfasermembran mit Vinyldimethylazlacton

2,56 g 1,8-Diazabicyclo[5,4,0]undec-7-en (DBU) und 32 ml Vinyldimethylazlacton werden in 160 ml Dimethylformamid gelöst. Ein Polyamidhohlfaserbündel (Polyamid 6, 64 Fäden; Durchmesser der Einzelfaser; innen 0,2 mm, außen 0,5 mm; mittlere Porenweite 0,5 µm) wird in eine 300 - 10 mm Chromatographiesäule SUPERFORMANCE® (Fa. E. Merck) gepackt und die obige Lösung bei Raumtemperatur mit einem Fluß von 2 ml/min 24 Stunden durch das Hohlfaserbündel im Kreis gepumpt. Anschließend wird das derivatisierte Hohlfaserbündel mit Dimethylformamid, Aceton, Essigsäureethylester und Aceton gespült und im Vakuumtrockenschrank bei 50 °C getrocknet.

### Beispiel 2: Bindung von γ - Globulin an eine mit Vinyldimethylazlacton aktivierte Polyamidhohlfasermembran

1 g γ-Globulin wird in 100 ml Tris-Puffer (50 mM, pH 7,4) gelöst und in der in Beispiel 1 beschriebenen Apparatur durch das nach Beispiel 1 derivatisierte Hohlfaserbündel bei Raumtemperatur im Kreis gepumpt (Fluß: 5 ml/min). Dabei wurden die Proteinkonzentration in der Lösung und deren Abnahme kontinuierlich UV-spektrophotometrisch bestimmt; nach zwei Stunden blieb die Proteinkonzentration in der umgepumpten Lösung konstant. Nach Auswaschen des Hohlfaserbündels mit Tris-Puffer (50 mM, pH 7,4) und 0,1 M Essigsäure wurde das im Hohlfaserbündel kovalent gebundene Protein bestimmt: 66,5 mg.

Ein Hohlfasermodul mit einer wie oben beschrieben mit γ-Globulin belegten Polyamidmembran kann für die Affinitätschromatographie zur Bindung von anti-y-Globulin-Antikörpern verwendet werden.

### Beispiel 3: Reaktion eines Polyamids mit Ethylendiamin

Zur Durchführung der Synthese wird ein Polyamidhohlfaserbündel aus Polyamid 6 in eine 300-10 mm Chromatographiesäule SUPERFORMANCE ® (Fa. E. Merck) gepackt. An diese Säule wird eine inerte Pumpe angeschlossen. Für die Umsetzung werden 10 Mol Ethylendiamin und 0,2 Mol 1-Ethyl-3-(3-dimethylaminopropyl)-carbodiimid (EDC) in 200 ml 0,1 M Natriumacetatpuffer (pH 4,7) gelöst und mit hoher Geschwindigkeit (5 ml/ min) bei Raumtemperatur 5 Stunden im Kreis gepumpt. Anschließend wird die derivatisierte Membran 1 M Phosphatpuffer pH 7 und mit Wasser neutral gewaschen.

### Beispiel 4: Umsetzung einer Polyamidhohlfasermembran mit Vinyldimethylazlacton

Die Umsetzung des nach Beispiel 3 aminomodifizierten Polyamids wird wie in Beispiel 1 beschrieben ausgeführt.

## Patentansprüche

1. Azlacton-derivatisiertes Polyamid erhältlich durch Umsetzung von Polyamiden mit einer Lösung enthaltend eine Azabicyclo-Verbindung und ein Vinylazlactonderivat der Formel I, 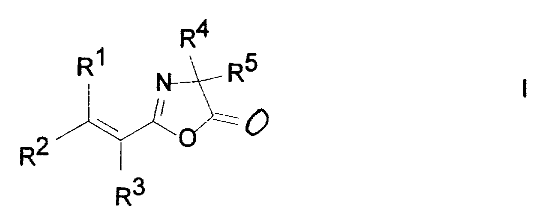 worin
R¹, R² und R³ unabhängig voneinander
H oder CH₃;
R⁴ und R⁵ unabhängig voneinander
H oder C₁- bis C₅- Alkyl
bedeuten.

2. Azlacton-derivatisiertes Polyamid nach Anspruch 1, dadurch gekennzeichnet, daß Carboxylgruppen des Polyamides vor der Umsetzung mit dem Vinylazlactonderivat der Formel I mit einer Diaminoverbindung umgesetzt werden.

3. Verwendung eines azlacton-derivatisiertem Polyamid nach einem der Ansprüche 1 oder 2 zur Bindung von aminogruppenhaltigen Verbindungen.

4. Trennmaterial erhältlich durch Umsetzung eines azlacton-derivatisierten Polyamides nach einem der Ansprüche 1 oder 2 mit einem aminogruppenhaltigen Separationseffektor.

5. Immobilisiertes Enzym erhältlich durch Umsetzung eines azlacton-derivatisierten Polyamides nach einem der Ansprüche 1 oder 2 mit einem Enzym.

## Claims

1. Azlactone-derivatized polyamide obtainable by reacting polyamides with a solution comprising an azabicyclo compound and a vinylazlactone derivative of the formula I 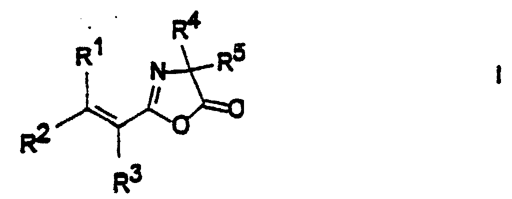 in which
R¹, R² and R³ independently of one another denote H or CH₃;
R⁴ and R⁵ independently of one another denote H or C₁- to C₅- alkyl.

2. Azlactone-derivatized polyamide according to Claim 1, characterized in that carboxyl groups of the polyamide are reacted with a diamino compound prior to the reaction with the vinylazlactone derivative of the formula I.

3. Use of an azlactone-derivatized polyamide according to one of Claims 1 or 2 for binding amino-containing compounds.

4. Separation material obtainable by reacting an azlactone-derivatized polyamide according to one of Claims 1 or 2 with an amino-containing separation effector.

5. Immobilized enzyme obtainable by reacting an azlactone-derivatized polyamide according to one of Claims 1 or 2 with an enzyme.

## Revendications

1. Polyamide dérivatisé par une azlactone, pouvant être obtenu par réaction de polyamides avec une solution contenant un composé azabicyclo et un dérivé de vinylazlactone de formule I 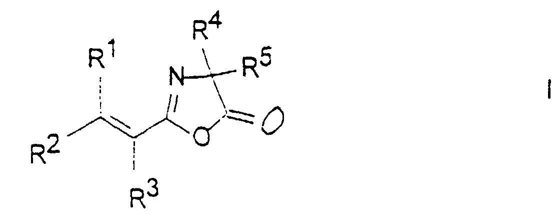 dans laquelle
R¹, R² et R³ représentent chacun indépendamment des autres H ou CH₃ ;
R⁴ et R⁵ représentent chacun indépendamment de l'autre H ou un groupe alkyle en C₁ à C₅.

2. Polyamide dérivatisé par une azlactone selon la revendication 1, caractérisé en ce que les groupes carboxyle du polyamide sont, avant la réaction avec le dérivé de vinylazlactone de formule I, mis à réagir avec un composé diamino.

3. Utilisation d'un polyamide dérivatisé par une azlactone selon l'une des revendications 1 ou 2, pour lier des composés contenant des groupes amino.

4. Matériau séparateur, pouvant être obtenu par réaction d'un polyamide dérivatisé par une azlactone selon l'une des revendications 1 ou 2 avec un effecteur de séparation contenant des groupes amino.

5. Enzyme immobilisée, pouvant être obtenue par réaction d'un polyamide dérivatisé par une azlactone selon l'une des revendications 1 ou 2 avec une enzyme.
